# EUROPÄISCHE PATENTSCHRIFT

(11) **EP 3 473 200 B1**
(45) Veröffentlichungstag und Bekanntmachung des Hinweises auf die Patenterteilung: **07.10.2020**
(21) Anmeldenummer: 18156706.6
(22) Anmeldetag: 14.02.2018
(51) Int. Cl.: A61B 18/14, A61B 17/00, A61B 5/0245, A61B 5/042, A61B 5/053, A61B 18/00

(54) **ABLATIONSKATHETER MIT MIKROELEKTRODE UND VERFAHREN ZUM HERSTELLEN EINES ABLATIONSKATHETERS**
ABLATION CATHETER WITH MICROELECTRODE AND METHOD FOR PRODUCING AN ABLATION CATHETER
CATHÉTER D'ABLATION MUNI DE MICRO-ÉLECTRODE ET PROCÉDÉ DE FABRICATION D'UN CATHÉTER D'ABLATION

(30) Priorität: 23.10.2017 DE 102017124651
(43) Veröffentlichungstag der Anmeldung: 24.04.2019
(73) Patentinhaber: VascoMed GmbH, 79589 Binzen (DE)
(72) Erfinder: Kaufmann, Ralf, 79540 Lörrach (DE)
(74) Vertreter: Keck, Hans-Georg

(56) Entgegenhaltungen:
- EP-A1- 3 009 092
- EP-A1- 3 040 043
- WO-A1-2015/183635
- WO-A1-2017/070559
- DE-A1- 10 008 918
- US-A1- 2014 081 111

## Beschreibung

Die Offenbarung betrifft einen Ablationskatheter mit einer Mikroelektrode sowie ein Verfahren zum Herstellen eines Ablationskatheters.

### Hintergrund

Das intrakardiale Mapping (z. B. durch Impedanzmessung zwischen Katheterelektroden) in der Kontaktzone einer Ablationselektrode eines elektrophysiologischen Katheters mit dem Herzgewebe ist bekannt. Das sogenannte "Micro-Mapping" mittels einer oder mehrerer Mikroelektroden an dem Katheter ist ein vielversprechender Ansatz, um für medizinische Diagnose- und Überwachungszwecke während der Ablation zur Behandlung von Herzrhythmusstörungen genutzt zu werden.

Das Dokument US 2004/0092806 A1 offenbart einen Ablationskatheter mit Mikroelektroden. Die metallischen Mikroelektroden in Form von Zylindern, Kegeln oder Pilzen sind in Bohrungen in der Ablationselektrode und von dieser elektrisch isoliert eingefügt und im innenliegenden Bereich des Ablationskatheters mit elektrischen Zuleitungen versehen.

Das Dokument US 2008/0243214 A1 offenbart ebenfalls einen Ablationskatheter mit Mikroelektroden. Die Mikroelektroden sind als Zylinder geformt und in Löchern am Katheterkopf angeordnet.

Das Dokument EP 3 015 064 A2 offenbart einen Ablationskatheter mit Vertiefungen zur Aufnahme von Mikroelektroden.

Dokument US20140081111A1 beschreibt einen Katheter, bei dem im distalen Bereich Mapping-Elektrodenpole auf der Ablationselektrode angeordnet sind.

Dokument EP3040043A1 beschreibt einen Katheter, bei dem auf einem porösen Substrat am distalen Ende Mikroelektroden und Zuleitungen angeordnet sind.

Die Dokumente EP3009092A1 und DE10008918A1 zeigen Elektrodenpole mit Mikroelektroden, wobei die Mikroelektroden durch die Wand der Elektrodenpole hindurch kontaktiert werden.

In Dokument WO2017070559A1 wird ein Katheter mit einem Mikroelektroden-Array beschrieben.

Die bekannten Mikroelektroden-Katheter-Konzepte benötigen für die Mikroelektroden einen relativ großen Platzbedarf auf der Ablationselektrode, insbesondere für die Befestigung, die elektrische Isolation und die Kontaktierung mit den elektrischen Zuleitungen. Bei gespülten Ablationskathetern beeinträchtigen die Gestaltung und die Montage der elektrischen Zuleitungsführung in hohem Maße die Führung der Spülleitungen und die Gestaltung der Spülkanäle.

### Zusammenfassung

Als Aufgabe kann angesehen werden, verbesserte Technologien für Ablationskatheter bereitzustellen. Insbesondere soll der Platzbedarf einer Mikroelektrode und deren Zuleitung verringert werden.

Es sind ein Ablationskatheter nach Anspruch 1 und ein Verfahren zum Herstellen eines Ablationskatheters nach Anspruch 14 offenbart. Weitere Ausführungsformen sind Gegenstand von abhängigen Ansprüchen.

Nach einem Aspekt ist ein Ablationskatheter bereitgestellt. Der Ablationskatheter weist einen Katheterschaft und eine an einem distalen Ende des Katheterschafts angeordnete Ablationselektrode auf. Des Weiteren sind eine Mikroelektrode und ein Leitungselement vorgesehen. Die Mikroelektrode ist an einer Oberfläche der Ablationselektrode angeordnet. Das Leitungselement ist mit der Mikroelektrode elektrisch leitend verbunden. Das Leitungselement ist von einem Isoliermaterial umgeben, so dass das Leitungselement von der Ablationselektrode elektrisch isoliert ist. Das Isoliermaterial ist mit dem Leitungselement zumindest abschnittsweise an der Oberfläche der Ablationselektrode angeordnet.

Nach einem weiteren Aspekt ist ein Verfahren zum Herstellen eines Ablationskatheters offenbart. Das Verfahren umfasst die folgenden Schritte: Bereitstellen eines Katheterschafts, Anordnen einer Ablationselektrode an einem distalen Ende des Katheterschafts, Anordnen einer Mikroelektrode an einer Oberfläche der Ablationselektrode, und Bereitstellen eines Leitungselements, das mit der Mikroelektrode elektrisch leitend verbunden ist. Das Leitungselement wird von einem Isoliermaterial umgeben, so dass das Leitungselement von der Ablationselektrode elektrisch isoliert ist. Das Isoliermaterial wird mit dem Leitungselement zumindest abschnittsweise an der Oberfläche der Ablationselektrode angeordnet.

Es können mehrere Mikroelektroden an der Ablationselektrode angeordnet sein, wobei jede der mehreren Mikroelektroden von der Ablationselektrode elektrisch isoliert sein kann. Jede der mehreren Mikroelektroden kann mit einem ausschließlich ihr zugeordneten Leitungselement verbunden sein. Es kann auch vorgesehen sein, dass mehrere Mikroelektroden mit einem gemeinsamen Leitungselement verbunden sind. Auch Mischformen sind möglich. Beispielsweise kann eine erste Gruppe von mehreren Mikroelektroden mit einem gemeinsamen ersten Leitungselement verbunden sein und eine zweite Gruppe von mehreren Mikroelektroden kann mit einem gemeinsamen zweiten Leitungselement verbunden sein. Die Mikroelektroden können vielfältig geformt sein, z. B. rund, oval oder halbkreisförmig. Die Mikroelektroden können auf der Oberfläche der Ablationselektrode in verschiedene Richtungen zeigen.

Die Mikroelektrode kann an einer Stirnfläche der Ablationselektrode oder an einer Mantelfläche der Ablationselektrode angeordnet sein. Wenn mehrere Mikroelektroden vorgesehen sind, können eine oder mehrere Mikroelektroden an der Stirnfläche der Ablationselektrode angeordnet sein. Ergänzend oder alternativ können eine oder mehrere Mikroelektroden an der Mantelfläche der Ablationselektrode angeordnet sein. Mehrere Mikroelektroden können an der Stirnfläche und/oder der Mantelfläche der Ablationselektrode gleichmäßig verteilt sein. Die Stirnfläche der Ablationselektrode kann auch als distales Ende der Ablationselektrode bezeichnet werden.

Als distales Ende des Katheterschafts wird das Ende verstanden, das bei der Verwendung des Katheters (beispielsweise während der Ablation) in den Körper des Patienten eingeführt wird. Das distale Ende des Katheterschafts mit der Ablationselektrode kann auch als Katheterkopf bezeichnet werden. Ein proximales Ende des Katheterschafts ist das Ende, das bei der Verwendung des Katheters außerhalb des Körpers verbleibt. An dem proximalen Ende des Katheterschafts kann ein Kathetergriff gebildet sein. An dem Kathetergriff kann eine Anschlusseinrichtung zum Anschluss der Ablationselektrode und der Mikroelektrode(n) an eine Steuervorrichtung vorgesehen sein.

Die Mikroelektrode und das mit dem Isoliermaterial umgebene Leitungselement sind in einer an der Oberfläche der Ablationselektrode gebildeten Aussparung angeordnet. Die Mikroelektrode und das die Mikroelektrode umgebende Leitungsmaterial können als in die Oberfläche der Ablationselektrode eingebettet angesehen werden. Die Aussparung kann an der Stirnfläche und/oder an der Mantelfläche der Ablationselektrode gebildet sein. Wenn mehrere Mikroelektroden vorgesehen sind, können mehrere Aussparungen an der Oberfläche der Ablationselektrode gebildet sein, derart, dass jede Mikroelektrode und/ oder das jeweilige Leitungselement in einer der mehreren Aussparungen angeordnet sind. Die Aussparung (oder ggf. die Aussparungen) kann eine Tiefe von 0,05 mm oder weniger (z. B. 0,03 mm oder 0,01 mm) haben.

Die Mikroelektrode und/oder das mit dem Isoliermaterial umgebene Leitungselement können in die Aussparung geklebt sein. Alternativ können die Mikroelektrode und/oder das Isoliermaterial mittels einer Klemmbefestigung, Umspannen, Aufschrumpfen oder Aufdehnen befestigt sein. Andere Möglichkeiten zur Befestigung der Mikroelektrode und/oder des mit dem Isoliermaterial umgebenen Leitungselements sind ebenfalls denkbar.

Die Mikroelektrode kann von der Ablationselektrode elektrisch isoliert sein, beispielsweise mittels des Isoliermaterials.

Das Isoliermaterial kann Polyimid (PI), Polyurethan (PUR), Polyether-Block-Amid (PEBA) oder ein Flüssigkristallpolymer (LCP - liquid crystal polymer) sein. Flüssigkristallpolymere sind einfach zu verarbeiten (auch bei kurzzeitig 100°C noch formstabil und beständig) und biokompatibel, was sie besonders zur Anwendung bei einem Ablationskatheter geeignet macht. Das Isoliermaterial kann als flexibles Folienmaterial bereitgestellt werden, z. B. als LCP-Folie oder eine Folie aus einem der anderen vorgenannten Materialien.

Die Mikroelektrode und/oder das mit dem Isoliermaterial umgebene Leitungselement können formschlüssig (oder passgenau) in der Aussparung angeordnet sein.

Das Leitungselement kann ein Metall (z. B. Kupfer) oder eine Metalllegierung enthalten oder aus einem Metall (z. B. Kupfer) bzw. einer Metalllegierung bestehen. Das Leitungselement kann als Leiterbahn ausgebildet sein. In einer Ausführungsform ist das Leitungselement als Kupferleiterbahn gebildet, die von einer flexiblen LCP-Folie zur elektrischen Isolierung umgeben ist.

Die Mikroelektrode (oder die Mikroelektroden) kann ein Metall (z. B. Kupfer) oder eine Metalllegierung enthalten oder aus einem Metall (z. B. Kupfer) bzw. einer Metalllegierung bestehen. Die Mikroelektrode kann beschichtet sein, beispielsweise mit einem Metall (z. B. Gold, Platin oder einem anderen galvanisierbaren biokompatiblen Metall) oder einer Metalllegierung. Die Mikroelektrode kann als flächige Mikroelektrode gebildet sein, wobei die Ausdehnung der Mikroelektrode in zwei Dimensionen (Fläche der Mikroelektrode) wesentlich größer ist als in die dritte Dimension (Höhe der Mikroelektrode). Die Ausdehnung in der Fläche (z. B. der Durchmesser der Mikroelektrode) kann 0,3 mm betragen. Beispielsweise kann die Mikroelektrode (oder ggf. die Mikroelektroden) als flächige (oder leicht gewölbte) Mikroelektrode aus Kupfer gebildet sein, die mit Gold beschichtet ist.

Die Dimensionen orientieren sich am Umfang und der axialen Länge der Ablationselektrode (z. B. Umfang U = 7 mm und Länge L = 3 bis 8 mm). Der Elektrodendurchmesser D der Mikroelektroden inklusive Isolationskanten ist maximal D = U/n, wobei n die Anzahl der Mikroelektroden ist. Wichtig ist der von den Mikroelektroden inklusive Isolationskanten abgedeckte (und somit nicht wirksame) Flächenanteil der Ablationselektrode. Dieser sollte etwa unter einem Drittel liegen. Die Umfangsformen der Mikroelektroden können so gestaltet sein, dass die freie Kontaktfläche der Ablationselektrode in jeder Roll- und Kippwinkelstellung mit dem Herzgewebe groß genug für eine effiziente Ablationsstromabgabe ist.

Es kann vorgesehen sein, dass die Mikroelektrode und/oder das mit dem Isoliermaterial umgebene Leitungselement einen glatten Abschluss mit der Oberfläche der Ablationselektrode bilden. Insbesondere kann ein Übergang von der Mikroelektrode und/oder dem Isoliermaterial zu der Oberfläche der Ablationselektrode frei von Unebenheiten oder Kanten sein.

Das mit dem Isoliermaterial umgebene Leitungselement kann von der Mikroelektrode bis zu einem distalen Ende des Katheterschafts an der Oberfläche der Ablationselektrode angeordnet sein und an dem distalen Ende des Katheterschafts in einen Innenbereich des Katheterschafts geführt sein. An dem distalen Ende des Katheterschafts ist ein Übergang vom Material der Ablationselektrode (in der Regel ein Metall, z. B. Gold oder Platin, oder eine Metalllegierung) zum Material des Katheterschafts (z. B. ein Kunststoff). An diesem Übergang kann das Leitungselement mit dem Isoliermaterial in den Innenbereich des Katheterschafts geführt werden.

In der Ablationselektrode kann eine Spülöffnung gebildet sein. Die Spülöffnung kann mit einer im Innenbereich des Katheterschafts angeordneten Spülleitung verbunden sein. Es können auch mehrere Spülöffnungen in der Ablationselektrode gebildet sein, die mit der Spülleitung verbunden sind. Anordnungen und Geometrien von Spülleitungen sind bekannt. Beispielsweise können die in dem Dokument EP 2 380 517 A1 offenbarten Ausführungsformen (insbesondere die in den Fig. 3B und 4B gezeigten Varianten) mit der vorliegenden Offenbarung kombiniert werden.

Die Mikroelektrode kann mit einem Loch gebildet sein und derart auf der Oberfläche der Ablationselektrode angeordnet sein, dass das Loch der Mikroelektrode zumindest abschnittsweise auf der Spülöffnung angeordnet. In diesem Fall kann eine Spülung durch das Loch in der Mikroelektrode erfolgen. Dies kann folgende Vorteile haben:
1. Erweiterte Flächenausnutzung und weitere Platzierungsmöglichkeiten für Mikroelektroden.
2. Schutz vor Überhitzung und daraus resultierende Thrombenbildung an den Materialübergängen: Isolationskante (Polymer) zur Mikro- und Ablationselektrode.

Die Mikroelektrode kann als Ringelektrode gebildet sein, welche die Ablationselektrode um deren Umfang herum umgibt.

Die Mikroelektrode kann als gezahnte Mikroelektrode gebildet sein.

Merkmale, die für die Mikroelektrode offenbart sind, können auf Ausführungsformen mit mehreren Mikroelektroden übertragen werden. Die Merkmale, welche im Zusammenhang mit dem Ablationskatheter offenbart sind, können in analoger Weise auf das Verfahren zum Herstellen des Ablationskatheters angewendet werden und umgekehrt.

### Beschreibung von Ausführungsbeispielen

Im Folgenden werden beispielhafte Ausführungsformen unter Bezugnahme auf Figuren näher erläutert. Es zeigen:
- Fig. 1: eine schematische Darstellung eines Ablationskatheters mit Mikroelektroden,
- Fig. 2: eine schematische Darstellung eines Katheterkopfs des Katheters aus Fig. 1,
- Fig. 3: einen Querschnitt des Katheterkopfs aus Fig. 2,
- Fig. 4: eine schematische Darstellung einer Mikroelektrode,
- Fig. 5: schematische Darstellungen von weiteren Ausführungsformen einer Mikroelektrode,
- Fig. 6: eine schematische Darstellung einer anderen Ausführungsform einer Mikroelektrode,
- Fig. 7: eine schematische Darstellung eines Katheterkopfs mit Mikroelektroden gemäß der Ausführungsform nach Fig. 6,
- Fig. 8: eine schematische Darstellung eines Katheterkopfs, wobei die Mikroelektroden als Ringelektroden ausgeführt sind,
- Fig. 9: eine schematische Darstellung eines Katheterkopfs, wobei die Mikroelektroden als gezahnte Mikroelektroden ausgeführt sind,
- Fig. 10: eine schematische Darstellung der Anschlüsse der Leitungselemente an die gezahnten Mikroelektroden aus Fig. 9, und
- Fig. 11 - 13: verschiedene Arten der Befestigung der Mikroelektroden an der Ablationselektrode.

Für gleiche Komponenten werden die gleichen Bezugszeichen verwendet.

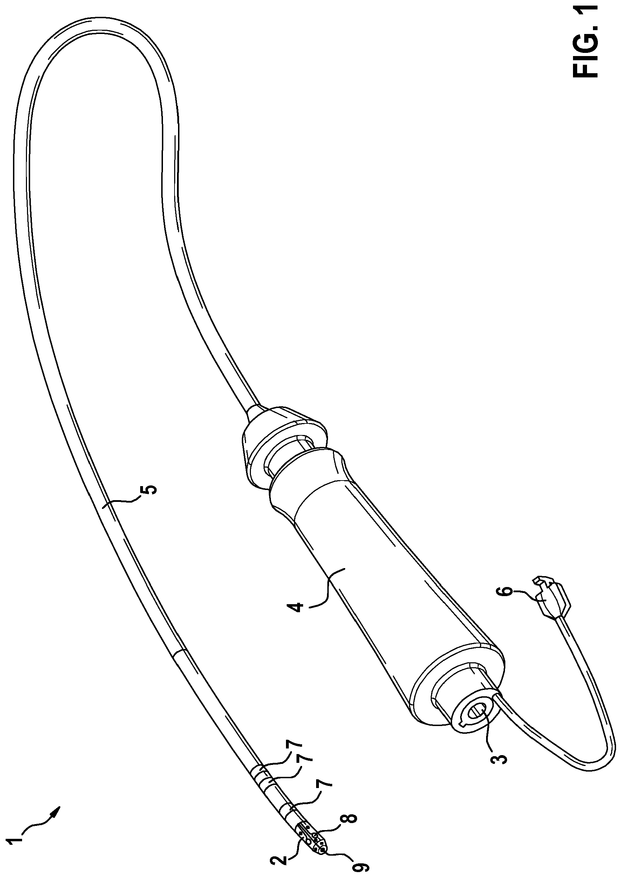

Fig. 1 bis 3 zeigen eine beispielhafte Ausführungsform eines Ablationskatheters 1 mit Mikroimpedanzmessfunktion, um beispielsweise Mikro-Mapping zu diagnostischen Zwecken und/oder zur Überwachung der Läsionsbildung während der Ablation durchzuführen.

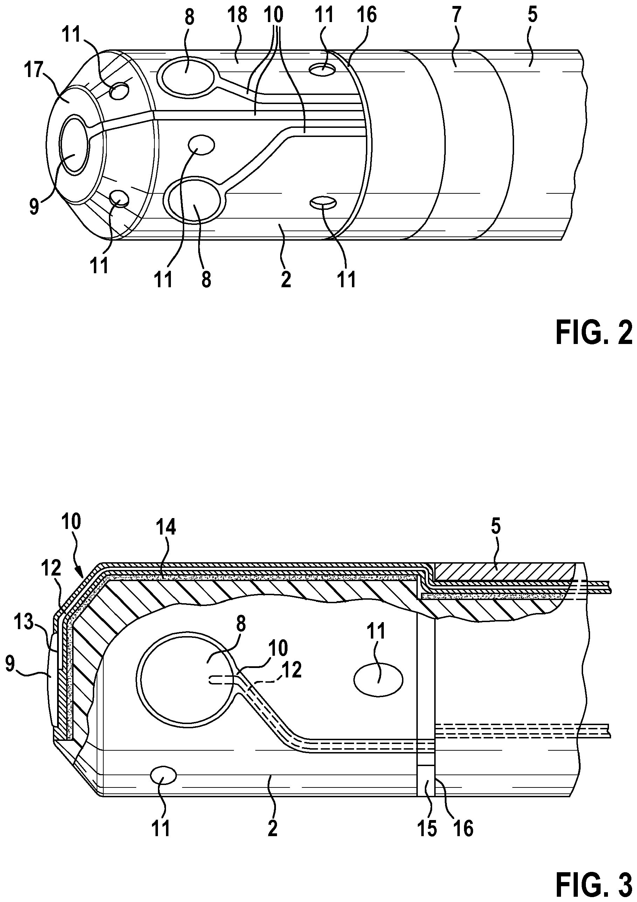

Fig. 1 stellt schematisch den Ablationskatheter 1 mit einer Ablationselektrode 2 dar. Die Ablationselektrode 2 ist an einem distalen Ende eines Katheterschafts 5 angeordnet. An der Ablationselektrode 2 sind laterale Mikroelektroden 8 auf einer Mantelfläche 18 (Fig. 2) der Ablationselektrode 2 und eine frontale Mikroelektrode 9 an einer Stirnfläche 17 (Fig. 2) der Ablationselektrode 2 angeordnet. Es können auch mehrere frontale Mikroelektroden an der Stirnfläche 17 angeordnet sein (nicht dargestellt). Proximal der Ablationselektrode 2 befinden sich drei Ringelektroden 7 für das herkömmliche Mapping-Verfahren. An einem proximalen Ende des Katheterschafts 5 ist ein Kathetergriff 4 angeordnet. Die Ablationselektrode 2, die lateralen Mikroelektroden 8, die frontale Mikroelektrode 9 und die Ringelektroden 7 sind über den Katheterschaft 5 durch den Kathetergriff 4 mit Polen in einem Stecker 3 verbunden. Der Stecker 3 stellt einen Anschluss für die Ablationselektrode 2, die lateralen Mikroelektroden 8, die frontale Mikroelektrode 9 und die Ringelektroden 7 und ggf. ein Thermoelement an eine Steuereinrichtung bereit. Ein Spülanschluss 6 für die Spülung der Ablationselektrode 2 ist aus dem Kathetergriff 4 herausgeführt.

Fig. 2 zeigt den Katheterkopf mit der Ablationselektrode 2 sowie einem Teil des Katheterschafts 5 und einer der Ringelektroden 7. Die Ablationselektrode 2 ist mit einer frontalen Mikroelektrode 9 und vier lateralen Mikroelektroden 8 ausgestattet. Die Mikroelektroden 8, 9 sind durch LCP-Folien 10 von der Ablationselektrode 2 elektrisch isoliert. Die LCP-Folien umgeben jeweils eine Leiterbahn, wobei jede Mikroelektrode 8, 9 mit einer Leiterbahn verbunden ist (nicht dargestellt). Die LCP-Folien 10 sind in Aussparungen in der Ablationselektrode 2 eingelassen. Die Tiefe der Aussparungen beträgt 0,05 mm. Die Mikroelektroden 8, 9 und die LCP-Folien 10 bilden einen glatten Abschluss mit der Oberfläche der Ablationselektrode 2. Die LCP-Folien 10 sind bis zu einem proximalen Ende des Ablationskatheters 2 an der Oberfläche des Ablationskatheters geführt. An dem proximalen Ende werden die LCP-Folien (mit den Leiterbahnen) in einen Innenbereich des Katheterschafts 5 geführt. Die LCP-Folien 10 können wie Papier gefaltet oder gebogen und somit leicht um eine Kante der Ablationselektrode 2 durch eine Klebung 15 in den Katheterschaft 5 geführt werden. Die LCP-Folien 10 sind so gestaltet, dass Spülöffnungen 11 gut umgangen werden und damit freigehalten sind.

Fig. 3 zeigt die Ablationselektrode 2 aus Fig. 2 so geschnitten, dass der Querschnitt einer Aussparung bis zur frontalen Mikroelektrode 9 sichtbar ist. Die LCP-Folie 10 ist hier mittels eines Klebers 14 für die Mikroelektrode 9 am Grund der Aussparung mit der Ablationselektrode 2 verbunden. Innerhalb der LCP-Folie 10 befindet sich eine Leiterbahn 12 (z.B. eine Kupferleiterbahn), die distal mit einer exponierten goldbeschichteten Elektrode 13 (z. B. eine Kupferelektrode) elektrisch leitend verbunden ist. Die Leiterbahn 12 ist gestrichelt gezeichnet, da diese vollständig von der LCP-Folie 10 umgeben (und daher nicht direkt sichtbar) ist. Nach proximal ist die Leiterbahn 12 elektrisch isoliert durch den Katheterschaft 5 und den Kathetergriff 4 geführt und schließlich mit einem entsprechenden Pol des Steckers 3 verbunden.

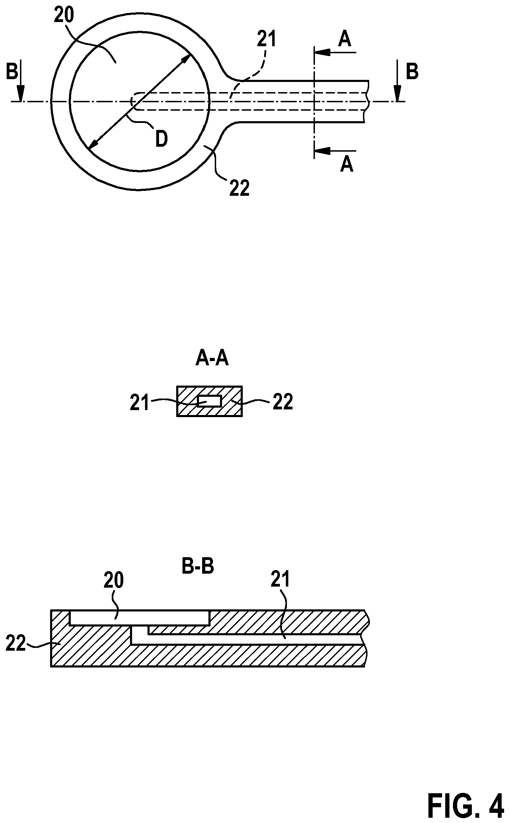

Fig. 4 zeigt eine schematische Darstellung einer Mikroelektrode 20. Die Mikroelektrode 20 hat in der gezeigten Ausführungsform eine kreisförmige Oberfläche mit einem Durchmesser D (z. B. 0,3 mm). Die Mikroelektrode 20 ist mittels eines Isoliermaterials 22 von der Ablationselektrode (nicht dargestellt) elektrisch isoliert. Das Isoliermaterial 22 kann beispielsweise eine LCP-Folie sein. Ein Leitungselement 21 (z. B. ein Kupferleiter) ist von dem Isoliermaterial umgeben, wie für den Querschnitt entlang der Linie A - A gezeigt ist. Des Weiteren ist ein Schnitt entlang der Linie B - B gezeigt. Die Mikroelektrode 20 ist an ihrer Unterseite mit dem Leitungselement 21 verbunden.

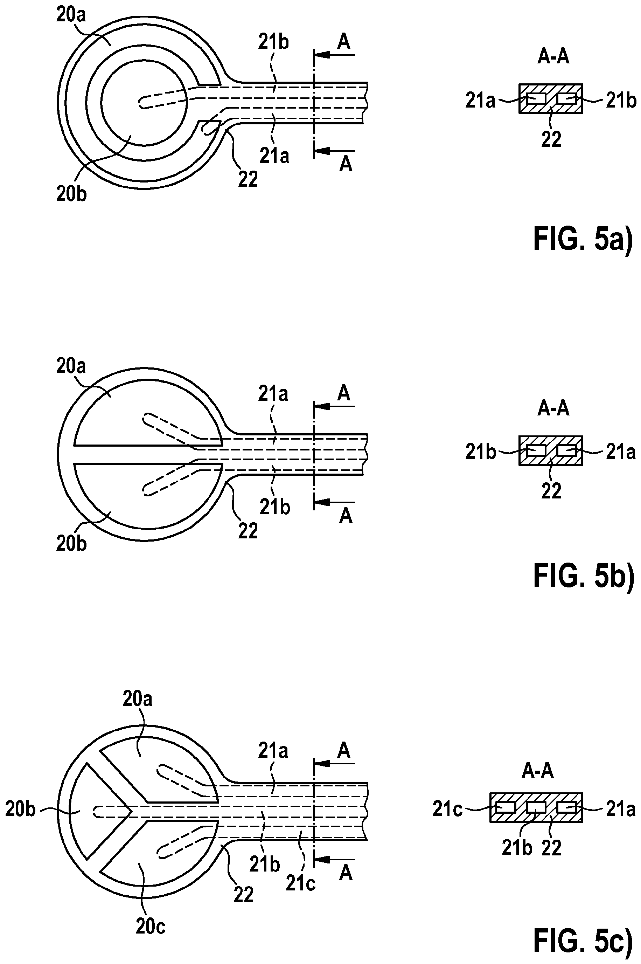

Fig. 5 zeigt schematische Darstellungen von Ausführungsformen einer mehrteiligen Mikroelektrode. Es ist jeweils auch ein Schnitt entlang der Linie A - A dargestellt.

Fig. 5 a) zeigt eine konzentrische Mikroelekrode mit zwei Segmenten 20a, 20b. Ein erstes Segment 20a bildet einen offenen odereinen geschlossenen Kreis, der ein zweites Segment 20b in Form einer Kreisfläche umgibt. Die beiden Segmente 20a, 20b sind mit Leitungselementen 21a, 21b verbunden. Die beiden Segmente 20a, 20b und die Leitungselemente 21b, 21b sind mit dem Isoliermaterial 22 von der Ablationselektrode (nicht dargestellt) isoliert.

In Fig. 5 b) ist eine zweiteilige Mikroelektrode mit zwei halbkreisförmigen Segmenten 20a, 20b dargestellt. Die beiden Segmente 20a, 20b sind mit Leitungselementen 21a, 21b verbunden. Die beiden Segmente 20a, 20b und die Leitungselemente 21b, 21b sind mit dem Isoliermaterial 22 von der Ablationselektrode (nicht dargestellt) isoliert.

Eine Mikroelektrode mit drei Segmenten 20a, 20b, 20c ist in Fig. 5 c) gezeigt. Die drei Segmente 20a, 20b, 20c sind mit Leitungselementen 21a, 21b, 21c verbunden. Die drei Segmente 20a, 20b, 20c und die Leitungselemente 21b, 21b, 21c sind mit dem Isoliermaterial 22 von der Ablationselektrode (nicht dargestellt) isoliert.

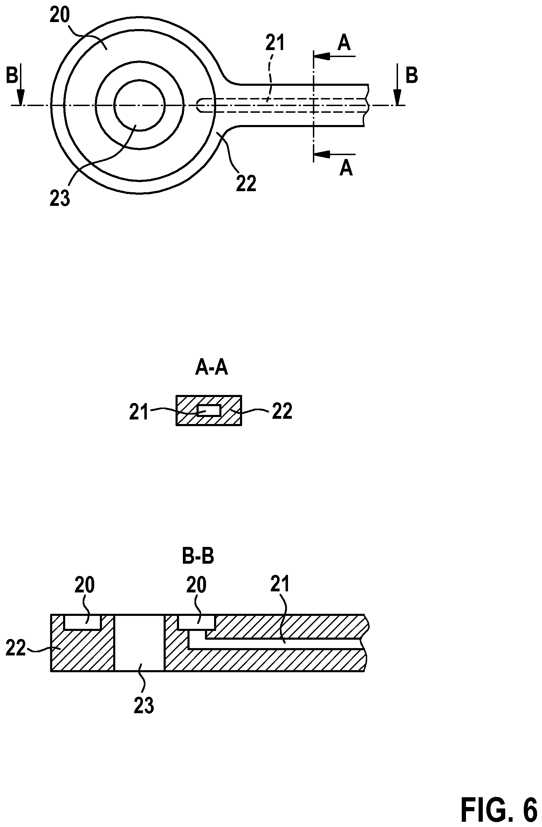

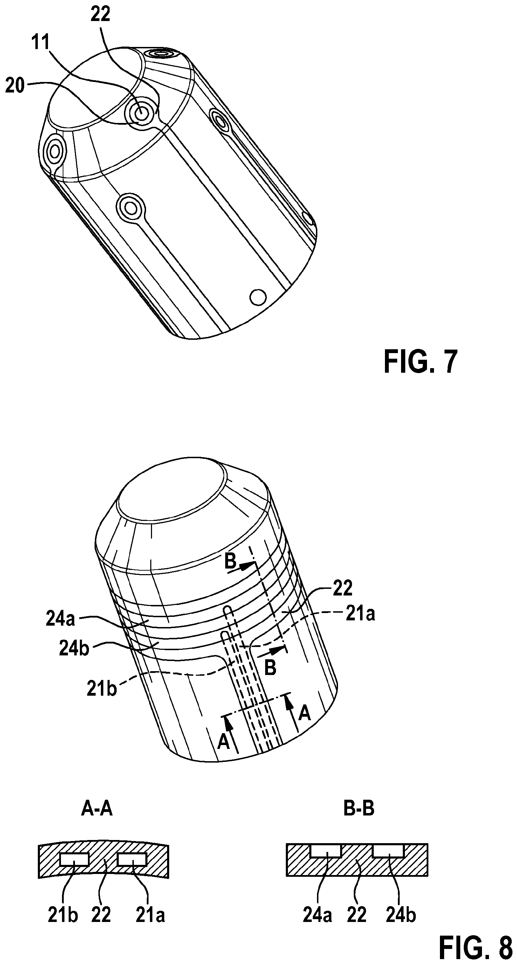

Fig. 6 zeigt eine Mikroelektrode 20 mit einem Loch 23. Die Mikroelektrode 20 ist mit dem Leitungselement 21 verbunden. Die Mikroelektrode 20 und das Leitungselement 21 sind mit dem Isoliermaterial 22 von der Ablationselektrode (nicht dargestellt) isoliert. Des Weiteren sind Schnitte entlang der Linien A - A und B - B dargestellt. Fig. 7 zeigt einen Katherkopf eines gespülten Katheters mit Mikroelektroden 20 nach Fig. 6. Die Spülöffnung 11 führt durch das Loch in der Mikroelektrode 20.

In Fig. 8 ist ein Katheterkopf mit Ring-Mikroelektroden 24a, 24b gezeigt. Die beiden Ring-Mikroelektroden 24a, 24b sind mit Leitungselementen 21a, 21b verbunden. Die beiden Ring-Mikroelektroden 24a, 24b und die Leitungselemente 21b, 21b sind mit dem Isoliermaterial 22 von der Ablationselektrode isoliert. Wie im Schnitt entlang der Linie A - A gezeigt ist, sind die Leitungselemente 21a, 21b vollständig von dem Isoliermaterial 22 umgeben. Der Schnitt entlang der Linie B - B zeigt die im Isoliermaterial 22 eingebetteten Ring-Mikroelektroden 24a, 24b.

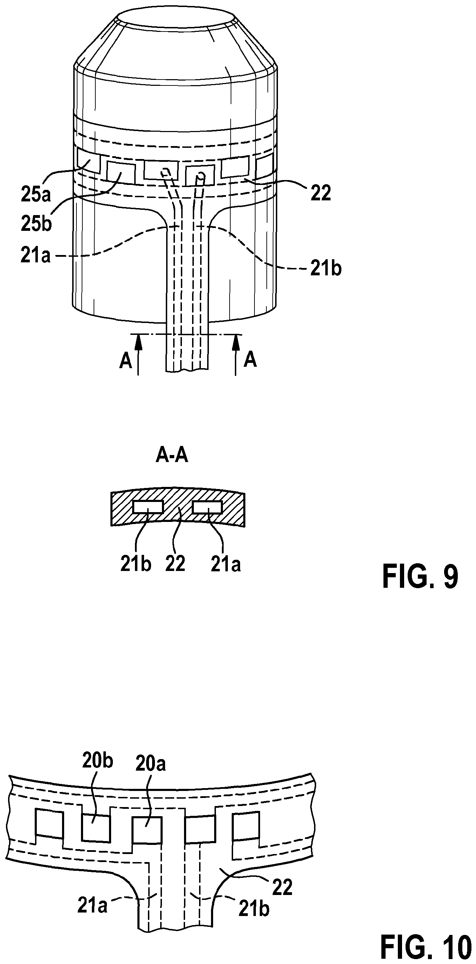

Fig. 9 zeigt einen Katheterkopf mit gezahnten Mikroelektroden 25a, 25b. Die beiden gezahnten Mikroelektroden 25a, 25b sind mit Leitungselementen 21a, 21b verbunden. Die beiden gezahnten Mikroelektroden 25a, 25b und die Leitungselemente 21b, 21b sind mit dem Isoliermaterial 22 von der Ablationselektrode isoliert. Wie im Schnitt entlang der Linie A - A gezeigt ist, sind die Leitungselemente 21a, 21b vollständig von dem Isoliermaterial 22 umgeben. Die Anschlüsse der gezahnten Mikroelektroden 25a, 25b an die Leitungselemente 21a, 21b ist in Fig. 10 dargestellt. Die beiden Leitungselemente 21a, 21b sind mit Anschlusselementen verbunden. Auf diese Art können die gezahnten Mikroelektroden mit lediglich zwei Leitungselementen 21a, 21b verbunden werden. Weitere Zahnformen sind möglich, wie beispielsweise dreieckige Zähne oder halbkreisförmige Zähne.

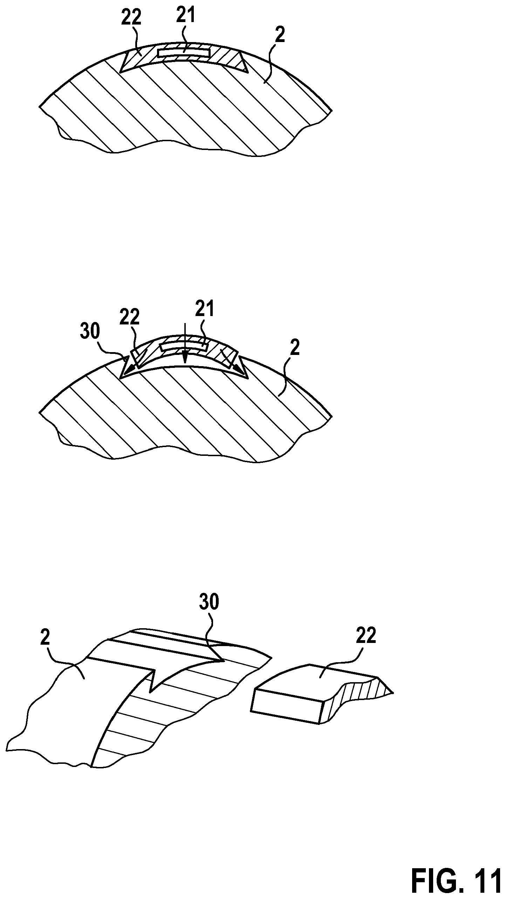

Fig. 11 zeigt eine Befestigung des Isoliermaterials 22 ohne Klebung. Stattdessen hakt das Isoliermaterial 22 hinter einer Kante 30 ein (Hinterschnitt-Klemmung). Das Isoliermaterial (und/oder die Mikroelektrode) kann in eine Aussparung eingeklipst werden, sodass sie hinter der Kante 30 verhakt (mittleres Bild von Fig. 11) oder sie kann in die Aussparung hinein geschoben werden (unteres Bild von Fig. 11).

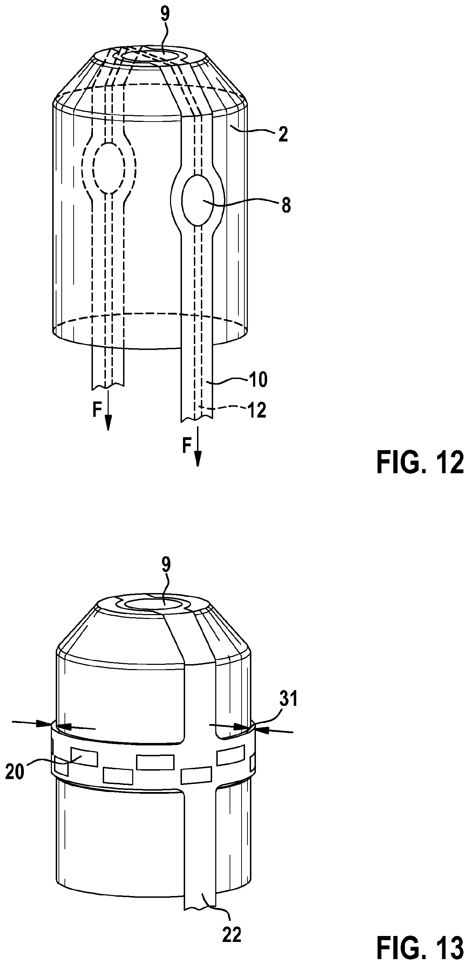

In Fig. 12 ist eine Befestigung mittels Umspannung in einer Nut dargestellt.

Fig. 13 zeigt eine Befestigung mittels Aufschrumpfen. Der Ablationskatheter ist hierbei kurzzeitig kühler als die Mikroelektroden 20 mit deren Zuleitung 21. Die Mikroelektroden mit den Zuleitungen und dem Isoliermaterial werden in einer Nut 31 angeordnet. Nach dem Abkühlen sitzen die Mikroelektroden fest auf der Ablationselektrode. Alternativ ist auch ein Aufdehnen der Mikroelektroden mit dem Isoliermaterial und den Zuleitungen und ein anschließendes Einrasten in der Nut möglich (nicht dargestellt).

Der Ablationskatheter nach den offenbarten Ausführungsformen kann die folgenden Vorteile aufweisen:
1. Die Mikroelektroden haben einen sehr geringen Platzbedarf an der Ablationselektrode, insbesondere für die Befestigung, die elektrische Isolation und die Kontaktierung mit den elektrischen Zuleitungen.
2. Die Gestaltung und die Montage der elektrischen Zuleitungsführung ist relativ einfach durch Kleben, Falten und Biegen zu bewältigen und beeinträchtigt nicht die Führung der Spülleitungen und der Spülkanäle in der Ablationselektrode.
3. Bei der Verwendung von mehreren Mikroelektroden kann gewährleistet sein, dass während der Ablation immer mindestens eine Mikroelektrode in Gewebekontakt ist.
4. Insbesondere die Ausführung eines Ablationskatheters mit partiellen LCP-Oberflächen ist biokompatibel und EO-sterilisierbar.
5. Elektrode mit Loch für bessere Flächenausnutzung und weitere Platzierungsmöglichkeiten für Mikroelektroden und Schutz vor Überhitzung und daraus resultierender Thrombenbildung an den Materialübergängen: Isolationskante (Polymer) zur Mikro- und Ablationselektrode.

Die in der Beschreibung, den Ansprüchen und den Figuren offenbarten Merkmale können für die Verwirklichung von Ausführungsformen sowohl einzeln als auch in beliebiger Kombination miteinander relevant sein.

### Bezugszeichenliste:

- 1: Ablationskatheter
- 2: Ablationselektrode
- 3: Stecker
- 4: Kathetergriff
- 5: Katheterschaft
- 6: Spülanschluss
- 7: Ringelektroden
- 8: Mikroelektrode lateral
- 9: Mikroelektrode frontal
- 10: LCP-Folie
- 11: Spülöffnung
- 12: Leiterbahn
- 13: Elektrode
- 14: Kleber für die Mikroelektrode
- 15: Klebung der Ablationselektrode mit dem Katheterschaft
- 16: distales Ende des Katheterschafts
- 17: Stirnfläche der Ablationselektrode
- 18: Mantelfläche der Ablationselektrode
- 20(a,b,c): Mikroelektrode
- 21(a,b,c): Leiterelement
- 22(a,b,c): Isoliermaterial
- 23: Loch
- 24(a,b): Ring-Mikroelektrode
- 25(a,b): gezahnte Mikroelektrode
- 30: Kante
- 31: Nut

## Patentansprüche

1. Ablationskatheter (1), mit
- einem Katheterschaft (5),
- einer Ablationselektrode (2), die an einem distalen Ende des Katheterschafts (5) angeordnet ist,
- einer Mikroelektrode (20; 24; 25), die an einer Oberfläche der Ablationselektrode (2) angeordnet ist, und
- einem Leitungselement (21), das mit der Mikroelektrode (20; 24; 25) elektrisch leitend verbunden ist,
wobei das Leitungselement (12; 21) von einem Isoliermaterial (10; 22) umgeben ist, so dass das Leitungselement (12; 21) von der Ablationselektrode (2) elektrisch isoliert ist,
**dadurch gekennzeichnet, dass**
die Mikroelektrode (20; 24; 25) und das mit dem Isoliermaterial (10; 22) umgebene Leitungselement (12; 21) in einer an der Oberfläche der Ablationselektrode (2) gebildeten Aussparung angeordnet sind.

2. Ablationskatheter (1) nach Anspruch 1, wobei die Mikroelektrode (20; 24; 25) und/oder das mit dem Isoliermaterial (10; 22) umgebene Leitungselement (12; 21) in die Aussparung geklebt sind.

3. Ablationskatheter (1) nach einem der vorhergehenden Ansprüche, wobei die Mikroelektrode (20; 24; 25) mittels des Isoliermaterials (10; 22) von der Ablationselektrode (2) elektrisch isoliert ist.

4. Ablationskatheter (1) nach einem der vorhergehenden Ansprüche, wobei das Isoliermaterial (10; 22) ein flexibles Folienmaterial ist.

5. Ablationskatheter (1) nach einem der vorhergehenden Ansprüche, wobei das Isoliermaterial (10; 22) ein Flüssigkristallpolymer ist.

6. Ablationskatheter (1) nach einem der vorhergehenden Ansprüche, wobei die Mikroelektrode (20; 24; 25) und/oder das mit dem Isoliermaterial (10; 22) umgebene Leitungselement (12; 21) einen glatten Abschluss mit der Oberfläche der Ablationselektrode (2) bilden.

7. Ablationskatheter (1) nach einem der vorhergehenden Ansprüche, wobei das mit dem Isoliermaterial (10; 22) umgebene Leitungselement (12; 21) von der Mikroelektrode (20; 24; 25) bis zu einem distalen Ende (16) des Katheterschafts (5) an der Oberfläche der Ablationselektrode (2) angeordnet ist und an dem distalen Ende (16) des Katheterschafts (5) in einen Innenbereich des Katheterschafts (5) geführt ist.

8. Ablationskatheter (1) nach einem der vorhergehenden Ansprüche, wobei in der Ablationselektrode (2) eine Spülöffnung (11) gebildet ist, und wobei die Spülöffnung (11) mit einer im Innenbereich des Katheterschafts angeordneten Spülleitung verbunden ist.

9. Ablationskatheter (1) nach Anspruch 8, wobei die Mikroelektrode (20) mit einem Loch (23) gebildet ist, und derart auf der Oberfläche der Ablationselektrode (2) angeordnet ist, dass das Loch (23) der Mikroelektrode (20) zumindest abschnittsweise auf der Spülöffnung (11) angeordnet ist.

10. Ablationskatheter (1) nach einem der Ansprüche 1 bis 8, wobei die Mikroelektrode (24) als Ringelektrode gebildet ist, welche die Ablationselektrode (2) um deren Umfang herum umgibt.

11. Ablationskatheter (1) nach einem der Ansprüche 1 bis 8, wobei die Mikroelektrode (25) als Mikroelektrode mit gezahntem Rand gebildet ist.

12. Verfahren zum Herstellen eines Ablationskatheters (1), mit folgenden Schritten:
- Bereitstellen eines Katheterschafts,
- Anordnen einer Ablationselektrode (2) an einem distalen Ende des Katheterschafts,
- Anordnen einer Mikroelektrode (20; 24; 25) an einer Oberfläche der Ablationselektrode (2), und
- Bereitstellen eines Leitungselements (12; 21), das mit der Mikroelektrode (20; 24; 25) elektrisch leitend verbunden ist,
wobei das Leitungselement (12; 21) von einem Isoliermaterial (10; 22) umgeben wird, so dass das Leitungselement (12; 21) von der Ablationselektrode (2) elektrisch isoliert ist, und wobei die Mikroelektrode (20; 24; 25) und das mit dem Isoliermaterial (10; 22) umgebene Leitungselement (12; 21) in einer an der Oberfläche der Ablationselektrode (2) gebildeten Aussparung angeordnet werden.

## Claims

1. An ablation catheter (1), comprising
- a catheter shaft (5);
- an ablation electrode (2) disposed at a distal end of the catheter shaft (5);
- a microelectrode (20; 24; 25) disposed on a surface of the ablation electrode (2); and
- a lead element (21) electrically conductively connected to the microelectrode (20; 24; 25);
the lead element (12; 21) being surrounded by an insulating material (10; 22) so that the lead element (12; 21) is electrically insulated from the ablation electrode (2),
**characterised in that**
the microelectrode (20; 24; 25) and the lead element (12; 21) surrounded by the insulating material (10; 22) are arranged in a recess formed on the surface of the ablation electrode (2).

2. The ablation catheter (1) according to claim 1, wherein the microelectrode (20; 24; 25) and/or the lead element (12; 21) surrounded by the insulating material (10; 22) are glued in the recess.

3. The ablation catheter (1) according to one of the preceding claims, wherein the microelectrode (20; 24; 25) is electrically insulated from the ablation electrode (2) by means of the insulating material (10; 22).

4. The ablation catheter (1) according to one of the preceding claims, wherein the insulating material (10; 22) is a flexible film material.

5. The ablation catheter (1) according to one of the preceding claims, wherein the insulating material (10; 22) is a liquid crystal polymer.

6. The ablation catheter (1) according to one of the preceding claims, wherein the microelectrode (20; 24; 25) and/or the lead element (12; 21) surrounded by the insulating material (10; 22) are flush with the surface of the ablation electrode (2).

7. The ablation catheter (1) according to one of the preceding claims, wherein the lead element (12; 21) surrounded by the insulating material (10; 22) is disposed on the surface of the ablation electrode (2) from the microelectrode (20; 24; 25) as far as a distal end (16) of the catheter shaft (5), and is guided into an interior of the catheter shaft (5) at the distal end (16) of the catheter shaft (5).

8. The ablation catheter (1) according to one of the preceding claims, wherein an irrigation opening (11) is formed in the ablation electrode (2), and wherein the irrigation opening (11) is connected to an irrigation line arranged in the interior of the catheter shaft.

9. The ablation catheter (1) according to claim 8, wherein the microelectrode (20) is formed with a hole (23), and is disposed on the surface of the ablation electrode (2) in such a way that at least sections of the hole (23) of the microelectrode (20) are disposed on the irrigation opening (11).

10. The ablation catheter (1) according to one of claims 1 to 8, wherein the microelectrode (24) is formed as a ring electrode that surrounds the ablation electrode (2) around its periphery.

11. The ablation catheter (1) according to one of claims 1 to 8, wherein the microelectrode (25) is formed as a microelectrode having a toothed edge.

12. A method for producing an ablation catheter (1), comprising the following steps:
- providing a catheter shaft;
- disposing an ablation electrode (2) on a distal end of the catheter shaft;
- disposing a microelectrode (20; 24; 25) on a surface of the ablation electrode (2); and
- providing a lead element (12; 21) that is electrically conductively connected to the microelectrode (20; 24; 25),
wherein the lead element (12; 21) is surrounded by an insulating material (10; 22) so that the lead element (12; 21) is electrically insulated from the ablation electrode (2), and wherein the microelectrode (20; 24; 25) and the lead element (12; 21) surrounded by the insulating material (10; 22) are arranged in a recess formed on the surface of the ablation electrode (2).

## Revendications

1. Cathéter d'ablation (1) doté
- d'une tige de cathéter (5),
- d'une électrode d'ablation (2), qui est disposée à une extrémité distale de la tige de cathéter (5),
- d'une micro électrode (20 ; 24 ; 25), qui est disposée au niveau d'une surface de l'électrode d'ablation (2), et
- d'un élément de conduction (21), qui est relié électriquement par conduction avec la micro électrode (20 ; 24 ; 25),
où l'élément de conduction (12 ; 21) est entouré par un matériau isolant (10 ; 22), de sorte que l'élément de conduction (12 ; 21) est isolé électriquement de l'électrode d'ablation (2),
**caractérisé en ce que**
la micro électrode (20 ; 24 ; 25) et l'élément de conduction (12 ; 21) entouré avec le matériau isolant (10 ; 22) sont disposés dans un renfoncement formé à la surface de l'électrode d'ablation (2).

2. Cathéter d'ablation (1) selon la revendication 1, dans lequel la micro électrode (20 ; 24 ; 25), et/ou l'élément de conduction (12 ; 21) entouré avec le matériau isolant (10 ; 22), sont collés dans le renfoncement.

3. Cathéter d'ablation (1) selon l'une des revendications précédentes, dans lequel la micro électrode (20 ; 24 ; 25) est isolée électriquement de l'électrode d'ablation (2) au moyen du matériau isolant (10 ; 22).

4. Cathéter d'ablation (1) selon l'une des revendications précédentes, dans lequel le matériau isolant (10 ; 22) est un matériau en film souple.

5. Cathéter d'ablation (1) selon l'une des revendications précédentes, dans lequel le matériau isolant (10 ; 22) est un polymère à cristal liquide.

6. Cathéter d'ablation (1) selon l'une des revendications précédentes, dans lequel la micro électrode (20 ; 24 ; 25), et/ou l'élément de conduction (12 ; 21) entouré avec le matériau isolant (10 ; 22), forment une finition lisse avec la surface de l'électrode d'ablation (2).

7. Cathéter d'ablation (1) selon l'une des revendications précédentes, dans lequel l'élément de conduction (12 ; 21) entouré avec le matériau isolant (10 ; 22) est disposé sur la surface de l'électrode d'ablation (2) en partant de la micro électrode (20 ; 24 ; 25) jusqu'à une extrémité distale (16) de la tige de cathéter (5) et est mené, au niveau de l'extrémité distale (16) de la tige de cathéter (5), dans une zone intérieure de la tige de cathéter (5).

8. Cathéter d'ablation (1) selon l'une des revendications précédentes, dans lequel un orifice de nettoyage (11) est formé dans l'électrode d'ablation (2), et dans lequel l'orifice de nettoyage (11) est relié avec une conduite de nettoyage disposée dans la zone intérieure de la tige de cathéter.

9. Cathéter d'ablation (1) selon la revendication 8, dans lequel la micro électrode (20) est formée munie d'un trou (23) et est disposée de telle manière sur la surface de l'électrode d'ablation (2) que le trou (23) de la micro électrode (20) est disposé au moins partiellement sur l'orifice de nettoyage (11).

10. Cathéter d'ablation (1) selon l'une des revendications 1 à 8, dans lequel la micro électrode (24) est conçue sous forme d'électrode annulaire, laquelle entoure l'électrode d'ablation (2) autour de sa périphérie.

11. Cathéter d'ablation (1) selon l'une des revendications 1 à 8, dans lequel la micro électrode (25) est conçue sous forme de micro électrode avec un bord dentelé.

12. Procédé de fabrication d'un cathéter d'ablation (1) avec les étapes suivantes :
- la mise au point d'une tige de cathéter,
- la disposition d'une électrode d'ablation (2) à une extrémité distale de la tige de cathéter,
- la disposition d'une micro électrode (20 ; 24 ; 25) sur une surface de l'électrode d'ablation (2), et
- la mise au point d'un élément de conduction (12 ; 21), qui est relié électriquement par conduction avec la micro électrode (20 ; 24 ; 25),
où l'élément de conduction (12 ; 21) est entouré par un matériau isolant (10 ; 22), de sorte que l'élément de conduction (12 ; 21) est isolé électriquement de l'électrode d'ablation (2), et dans lequel la micro électrode (20 ; 24 ; 25) et l'élément de conduction (12 ; 21) entouré avec le matériau isolant (10 ; 22) sont disposés dans un renfoncement formé à la surface de l'électrode d'ablation (2).
